# EUROPEAN PATENT APPLICATION

(11) **EP 1 118 331 A1**
(43) Date of publication of application: **25.07.2001**
(21) Application number: 00400170.7
(22) Date of filing: 21.01.2000
(51) Int. Cl.: A61K 39/00, A61K 47/48, A61P 31/00, A61P 35/00, A61P 37/04

(54) **Method for enhancing the presentation of exogenous antigen by human antigen-presenting cells and opsonized micro particle complexes for applying this method**

(71) Applicant: I.D.M. IMMUNO-DESIGNED MOLECULES, 75011 Paris (FR); INSTITUT NATIONAL DE LA SANTE ET DE LA RECHERCHE MEDICALE (INSERM), 75654 Paris Cédex 13 (FR); CENTRE NATIONAL DE LA RECHERCHE SCIENTIFIQUE (CNRS), 75794 Paris Cedex 16 (FR)
(72) Inventor: Leserman, Lee, 13007 Marseille (FR); Nardin, Alessandra, F-75015 Paris (FR); Abastado, Jean-Pierre, F-75015 Paris (FR); Machy, Patrick, F-13009 Marseille (FR); Serre, Karine, F-13001 Marseille (FR); Bartholeyns, Jacques, F-49730 Turquant (FR)
(74) Representative: Grosset-Fournier, Chantal Catherine

(57) **Abstract**

The invention relates to an opsonized micro-particle complex comprising :
- a micro-particular vector encapsulating at least one antigen
- and at least one antibody or fragment thereof,
with said antibody being a human or humanized antibody or an antibody binding to human FcR with substantially the same affinity and avidity as the ones of a human antibody and with said antibody or fragment thereof having the carboxy terminal end of its Fc portion external with respect to the opsonized micro particle complex.

## Description

The present invention concerns a method for enhancing the presentation of exogenous antigen by human antigen-presenting cells. It also concerns the opsonized micro-particle complexes used for applying this method. The present invention also concerns the use of these opsonized micro-particle complexes for the preparation of a vaccine.

It is normally accepted that exogenous antigens are presented by antigen presenting cells (APC) to CD4+ T cells through MHC class II pathway, while endogenous antigens are presented to CD8+ cells through MHC class I pathway. In fact, the immune response to exogenous antigens depends also on the nature of the antigen, the type of APC and the pathway of internalisation of the antigen in endocytic compartments of the APC. Particulate antigens, such as micro particles, apoptotic bodies, liposomes, cell debris, bacterial or viral particles, can be taken up by APC by pinocytosis, macropinocytosis (fluid phase endocytosis), receptor dependent endocytosis or phagocytosis. The access of the particulate antigen to both intracellular MHC classes I and II depends on the intracellular transfer mechanisms. Uptake and processing of exogenous Ag is well understood for class II presentation but the mechanisms of recognition and passage into the cytosol of exogenous Ag, which become class I restricted is less well known.

Mouse bone marrow derived antigen presenting cells can recognize opsonized micro-particle complexes by virtue of their receptors of the Fc portion of IgG (FcγR). These opsonized micro-particle complexes may be efficiently taken up by FcγR and initiate T cell response to class II associated antigens (Serre K., Machy P., Grivel J-C., Jolly G., Brun N., Barbet J. & Leserman L. "Efficient presentation of multivalent antigens targeted to various cell surface molecules of dendritic cells and surface Ig of antigen-specific B cells" J. Immunol. 1998, 161, 6059-6067).

In murine models, it has been observed that antigen-IgG complexes targeting the antigen to FcγR promotes antigen presentation and maturation of dendritic cells (Regnault A., Lankar D., Lacabanne V., Rodriguez A., Théry C., Rescigno M., Saito T., Verbeek S., Bonnerot C., Ricciardi-Castagnoli P. & Amigorena S. "Fcγ receptor-mediated histocompatibility complex Class-I restricted antigen presentation after immune complex internalisation" J. Exp. Med. 1999, 189(2), 371-380).

Human immature dendritic cells (DCs) present a very high phagocytic activity. They do pinocytose soluble antigens, for example serum albumin, they phagocytose yeast particles in a mannose receptor dependant way (Boyer A., Andreu G., Romet-Lemonne J-L., Fridman W-H. & Teillaud J-L. « Generation of phagocytic MAK and MAC-DC for therapeutic use: Characterisation and *in vitro* functional properties » Exp. Hematol. 1999, 751-761), they phagocytose tumour cells and tumour apoptotic bodies. Human mature dendritic cells (DC) present surface molecules different from those of murine dendritic cells (C. Bonnerot & S. Amigorena "Murine low-affinity receptors for the Fc portion of IgG. Roles in cell activation and ligand internalisation" Receptors and Channels, 1993, vol.1, 73-79). Human DCs present very low expression of Fc receptors on their membrane. They express very little CD64 (FcγRI) and CD16 (FcγRIII) and have an activator form of CD32 (FcγRII) absent in murine cells, which express only an inhibitory form of CD32 (C. Bonnerot & S. Amigorena "Murine low-affinity receptors for the Fc portion of IgG. Roles in cell activation and ligand internalisation" Receptors and Channels, 1993, vol.1, 73-79. Data obtained by targeting murine high affinity receptors on DCs cannot therefore be simply extrapolated to human antibodies and human DCs expressing different receptor forms.

The present invention aims at providing a very original and efficient method for targeting an exogenous antigen to antigen presenting cells.

Another aim of the invention is to provide a method for targeting an exogenous antigen to antigen presenting cells, allowing antigen internalisation and presentation via both MHC class I and MHC class II presentation pathways.

Another aim of the invention is to provide a very high efficient method, as only a very small quantity of antigen is necessary for the induction of an efficient stimulation of CD4+ and CD8+ T cells by the antigen presenting cells.

Another aim of the invention is to provide opsonized micro particle complexes encapsulating an antigen, which can be used as a vaccine.

All these aims have been achieved by the invention, which in its broadest embodiment consists in an opsonized micro particle complex comprising:
- a micro-particular vector encapsulating at least one antigen
- and at least one antibody or fragment thereof,
with said antibody being a human or humanized antibody or an antibody binding to human FcR with substantially the same affinity and avidity as the ones of a human antibody and with said antibody or fragment thereof having the carboxy terminal end of its Fc portion external with respect to the opsonized micro particle complex.

It has been shown that antigen presentation by antigen-specific murine dendritic cells was more efficient for antigen encapsulated in cell surface targeted liposomes, and particularly in liposomes coated with antibodies that bind to Fc receptor, than for free antigen taken up by the same cell. Because of the differences existing between the surface molecules expressed by the murine and the human, and particularly the differences between the Fcγ receptors, it was not expected that human antigen presenting cells would have a markedly increase presentation of antigens via targeting of Fc receptors.

The expression "micro-particular vector" designates a vector able to encapsulate at least one antigen.

The expression "humanized antibody" designates a non-human antibody modified so as to be non-inducer of an immune response when administered to a human.

The expression "substantially the same affinity" indicates that the different antibody / FcR complexes are characterized by dissociation constants (K_{D}) of the same order of magnitude. The affinity can be determined according to binding tests and determination of the affinity by the Scatchard method. (Immunology : Roitt et al. 4° ed. De Boeck Université, 1997)

The expression "substantially the same avidity" indicates that the different antibody / FcR complexes are characterized by affinity and number of binding sites substantially of the same order of magnitude. The avidity can be determined according to the measure of a global intensity of fixation of the antigen to the antibody. (Immunology : Roitt et al. 4° ed. De Boeck University, 1997)

The antibody fragment can be characterized in that it possesses at least its constant part, the FcR binding domain of the antibody.

The expression "the carboxy terminal end of its Fc portion is external with respect to the opsonized micro particle complex" designates the constant part of the antibody having its end exposed and free for fixation to Fc receptor.

In an advantageous embodiment, the invention relates to an opsonized micro particle complex, wherein the antibody is bound to the micro-particular vector through its variable portion.

The expression "the antibody is bound to the micro-particular vector through its variable portion" designates an antibody the variable domain of which is directed to the micro-particular vector and the Fc domain of which is directed externally relatively to the micro particular vector.

According to an another advantageous embodiment, in the opsonized micro particle complex of the invention the micro-particular vector encapsulating the antigen bears determinants and the antibody is bound to the micro-particular vector through its variable portion specific of said determinants.

The expression "the micro-particular vector bears determinants" means that the vector presents molecules liable to be recognized by antibodies.

One of the interests of the invention lies in the fact that anti-determinant antibodies according to the invention *in vivo* deliver opsonized micro-particle complexes to tissues in an FcR-dependent manner, whereas the usual antigen-antibodies complexes may be unstable *in vivo,* because of the dissociation of the antigen/antibody complex.

Furthermore, in classical methods, when antigens are directly bound to anti-antigen antibodies for targeting purposes, the processing of the antigen may be altered or result in modification of its presentation, depending on the site on the antigen to which the antibody binds, possibly resulting in the failure to present critical peptides. The use of the opsonized micro particle complexes according to the invention, with antibodies binding determinants on the micro particles, does not have this drawback.

Another characteristic of the invention is that opsonized micro particle complexes are formed by the association of a micro particular vector bearing determinants and of antibodies binding to the determinant, the antibodies covering the micro particle and having their Fc part pointing externally. Therefore, the Fc part of the antibodies targets the antigen presenting cells and the targeting is not achieved by the variable part of the antibodies, as it is usually seen.

Another characteristic of the invention is that there is no formation of an opsonized micro particle complex between the antigen of interest and the targeting antibodies, but rather the antibodies bind specifically to a determinant located on the surface of the micro particular vector. Thus, the structure of the antigen itself may be unknown, and there is no need for an antibody directed against each antigen to be available, but only a specific antibody of a determinant is needed.

Anti-determinant antibodies taking part in the opsonized micro particle complexes of the invention are used according to the determinant associated to the micro particular vector. The isotype of the antibodies used to form the opsonized micro particle complexes may be chosen according to the known affinity of certain antibodies isotypes for the targeted Fc receptors. In a particular application of the invention, the antibodies can be natural anti-sugar antibodies present in human blood plasma.

In another advantageous embodiment, the opsonized micro particle complex of the invention is such that the antibody is directly linked to the micro-particular vector by its Fc portion, with the carboxy terminal end of the Fc portion being external with respect to the opsonized micro particle complex.

The expression "the antibody is directly linked to the micro-particular vector directly by its Fc portion" designates a complex made of micro particular vector on which the Fc portion of an antibody is covalently bound, with the carboxy terminal end of the Fc portion being external relatively to the micro particular vector.

In another advantageous embodiment, the opsonized micro particle complex of the invention is such that the micro-particular vector is a liposome or a micro particle, advantageously having a size of about 20 to about 1000 nm.

A micro-particular vector (other than a liposome) can designate other synthetic micro-particles of similar size.

Advantageously, in the opsonized micro particle complex of the invention the micro-particular vector contains at least one destabilizing agent for the membrane of the endocytic vesicle, for the improvement of the delivery of the antigen contained in said micro-particular vector, with said destabilizing agent being, for instance, proteins, peptides or lipids, of viral or synthetic origin.

The expression "destabilizing agent" designates an agent with specific sensibility properties, when inserted into the liposome membranes, this agent contributes to the destabilization of the liposome membranes.

pH sensitive formulations of liposomes can be done as described in Connor J., Yatvin M.B. and Huang L. "pH-sensitive liposomes: acid-induced liposome fusion" Proc. Natl. Acad. Sci., USA, 1984, 81, 1715-1718 ; Connor J. and Huang L., "pH sensitive liposomes as an efficient and target specific carrier for anti-tumor drugs" Cancer Res., 1986, 46, 3431-3435 ; Slepushkin V.A., Simoes S., Dazin P., Newman M.S., Guo L.S., Pedroso de Lima M.C. and Duzgunes N. "Sterically stabilized pH-sensitive liposomes. Intracellular delivery of aqueous contents and prolonged circulation in vivo" J. Biol. Chem., 1997, 272, 2382-2388.

Some liposomes formulations are based on acid-dependent titration of phosphatidylethanolamine. When it is present as a major phospholipid component of liposomes, it forms bilayers at neutral pH, but when protonated at acid pH undergoes a phase transition to micellar form, resulting in the breakdown of the liposomes and subsequent release of contents.

Fusion proteins from viruses, such as hemagglutinin from influenza virus, may be incorporated into the membranes of liposomes : these are so-called "virosomes". When these virosomes enter into acidic compartments they fuse with the membrane of endocytic vesicles and deliver their contents into the cytosol (Bron R., Ortiz A. and Wilshut J. "Cellular cytoplasmic delivery of a polypeptide toxin by reconstituted influenza virus envelopes (virosomes)" Biochemistry 1994, 33, 9110-9117).

A new lipid formulation has been optimized for both hyperthermic temperatures (39 to 40°C) that are readily achievable in the clinic, and rapid release of drugs. A highly bilayer compatible lysolipid, 1-palmitoyl-2-Hydroxy-glycero-3-Phosphocholine (MPPC), is incorporated into gel phase liposomes composed of 1,2 Dipalmitoyl-Glycero-3-Phosphocholine (DPPC). This compositional modification of the liposomes achieves a significantly enhanced release of entrapped liposome contents at mild hyperthermic temperatures between 39 and 40°C as compared to pure DPPC alone, which released only 20% of contents over a broader range of 40-45°C (Anyarambhatla G.R. and Needham D. "Enhancement of the phase transition permeability of the DPPC liposomes by incorporation of MPPC: a new temperature sensitive liposome for use with mild hyperthermia" J. Liposome Research, 9, 491-506.)

In an advantageous embodiment, in the opsonized micro particle complex of the invention, the determinant is a peptide, a polypeptide, a sugar molecule, DNP or an other determinant.

In another advantageous embodiment, in the opsonized micro particle complex of the invention, the antibody is a human or humanized antibody.

In another advantageous embodiment, in the opsonized micro particle complex of the invention, the antigen is a tumor antigen or an antigen relevant in auto immune or infectious diseases or an allergenic antigen and preferably a combination of tumor antigens.

The expression "antigen relevant in auto immune or infectious diseases" means an antigen documented in the pathology of these diseases.

An interest of the invention is that the opsonized micro-particle complexes can encapsulate one or many different antigens, all of which benefit from the targeting of the anti-determinant antibody. The antigens can be of tumor origin; they also can be of bacterial, viral autogenic or allogenic origin.

In another advantageous embodiment, in the opsonized micro-particle complex of the invention, the antigen-presenting cell is a dendritic cell.

Antigen presenting cells used for the method according to the invention may be from human or other mammal origin. These cells can be dendritic cells, and particularly monocyte-derived immature dendritic cells. Immature DCs phagocytose particulate antigens very effectively and are CD83 negative, whereas mature DCs have lost the phagocytic capacity and are CD83 positive.

The invention also relates to a combined preparation containing as active substance the following individual components, in the form of a kit of parts:
- a micro-particular vector encapsulating at least one antigen
- at least one antibody or fragment thereof, with said antibody being a human or humanized antibody or an antibody binding to human FcR with substantially the same affinity and avidity as the ones of a human antibody, and being liable to bind to said micro-particular vector, in such a way that the antibody or fragment thereof has the carboxy terminal end of its Fc portion which remains free,
- possibly human antigen presenting cells bearing Fc receptors, liable to bind to the above-mentioned free Fc portion of the antibody or fragment thereof, for the simultaneous, separate or sequential use, in the vaccination against cancer, infectious or autoimmune diseases.

According to an advantageous embodiment of the invention,
- the micro-particular vector, the antibody and the antigen-presenting cell are injected simultaneously, in a complexed or an independent form.
- the micro-particular vector and the antibody are injected simultaneously, in a complexed or an independent form
- the micro-particular vector is injected separately and natural antibodies present in the blood serum (for example anti-sugar antibodies) rapidly cover it.

The invention also relates to a ternary complex between the opsonized micro particle complex as above-defined, and a human antigen presenting cell bearing Fc receptors, wherein the opsonized micro particle complex is bound to the antigen presenting cell Fc receptor through the carboxy terminal end of Fc portion of the antibody.

The expression " the opsonized micro particle complex is bound to the antigen presenting cell Fc receptor through the carboxy terminal end of Fc portion of the antibody" means that the Fc part of the antibody bound to the micro particular vector binds to the FcR on the surface of the antigen presenting cell.

The invention also relates to the use of an opsonized micro particle complex, as above defined, or of a ternary complex as above defined as a drug, particularly as a vaccine.

The invention also relates to a vaccine comprising as active substance an opsonized micro particle complex as above defined, or a ternary complex as above defined, possibly in association with a pharmaceutically acceptable vehicle.

The quantity of antigen that is to be injected in this case can range from 10⁻⁶ to 10⁻¹⁰ mole and advantageously from 10⁻⁸ mole to 10⁻¹⁰ mole for an injection to a child or an adult patient.

The invention also relates to the use of an opsonized micro particle complex as above defined, or of a ternary complex as above-defined, for the preparation of a vaccine against cancer, infectious or auto-immune disease.

The invention also relates to a method for *in vitro,* or *in* vivo, or *ex vivo* targeting antigens to human antigen presenting cells allowing antigen presentation via MHC class I pathway, comprising the step of contacting an opsonized micro particle complex as above-defined, with human antigen presenting cells, to form a ternary complex between the opsonized micro-particle complex and said human antigen presenting cells.

The invention also relates to a method for *in vitro,* or *in vivo,* or *ex vivo* targeting antigens to human antigen presenting cells allowing antigen presentation via MHC class I pathway, comprising the step of contacting a micro-particular vector encapsulating at least one antigen, at least one antibody or fragment thereof, with said antibody being a human or humanized antibody or an antibody binding to human FcR with substantially the same affinity and avidity as the ones of a human antibody and liable to bind to the micro-particular vector in such a way that the carboxy terminal end of its Fc portion is external with respect to the opsonized micro particle complex, and human antigen presenting cells, to form a ternary complex between the micro-particular vector, the antibody and the human antigen presenting cells.

It is to be noted that each of the above-mentioned contacts can occur *in vitro*, as well as *in vivo*, or *ex vivo*.

According to an advantageous embodiment, in the method of the invention, the antigen presentation via MHC class II is also involved.

According to another advantageous embodiment, in the method of the invention, the stimulation of human CD8 + T cells specific for exogenous antigen is involved.

The method of the invention thus enables the antigen presenting cells to present antigen to CD4+ T cells, via the MHC class II pathway, and to the CD8+ T cells via the MHC class I pathway.

The induction of the stimulation of CD4+T and CD8+T cells is efficient because, by way of illustration, 3-log10 lower encapsulated antigen concentrations induces the same level of Ag specific T cells stimulation as the antigen presented in the absence of the opsonized micro particle complexes. The advantage of using *in vivo* the opsonized micro particle complexes according to the invention is that the effective antigen doses can easily be reached and are expected to be too low to have any pathological consequences.

Other advantages of the invention are described below.

Opsonized micro particle complexes according to the invention may contain not only antigens but also some substances modulating the effect of these antigens in a stimulatory way, for example poly-IC, or in an inhibitory way, for example anti-sense nucleic acid or glucocorticoïds.

The present invention shows that micro particles, such as liposomes or other synthetic particles containing the antigen, targeted to the Fc receptor via antibodies with the variable high affinity part fixed to the micro particle and the constant Fc part pointing outside the particle, increases by several logs the efficiency of Ag presentation to T cells.

The binding of Ag to human dendritic cells (DC) is markedly enhanced for particulate opsonized micro particle complexes rather than for Ag in a soluble form. The binding to DCs of liposomes covered with antibodies and containing the Ag is blocked by the addition of an anti-FcγRII mAb ; this result indicates that an FcγRII mediates the binding of the opsonized liposome, which is a human stimulatory CD32 form of Fcγ receptor not existing in mice. The binding of liposomes to the DCs is followed by processing via internalisation of the FcRs and results in increased presentation of the Ag to the T cells. In particular, a very effective stimulation of antigen- specific CD8 + T cells is seen.

The evidence of the immune stimulation is the secretion of IFN gamma by Ag specific T cells.

The encapsulation of an antigen in a micro particular vector bearing determinants can be done according to a method known by a man skilled in the art. For example, the micro particular vector containing the antigen can be a liposome, formed by exposing lipids to an aqueous solution containing the antigen. The determinants may be bound to one part of the lipids used for the constitution of the liposomes.

The antigen-containing micro-particular vectors are incubated with antibodies that specifically bind to the determinants. The opsonized micro particle complexes thus formed are characterised by the fact the antibodies bind to determinants and not directly to exogenous antigens. Furthermore, the targeting of the antigen presenting cells is mediated by the Fc part of the antibodies and not by their variable part.

The opsonized micro particle complexes formed between the micro particles and the antibodies are incubated with antigen presenting cells in conditions allowing the binding of the opsonized micro particle complex on FcR located on the surface of the cells. This binding is followed by the internalisation of the complexes by the cells.

### Description of the figures

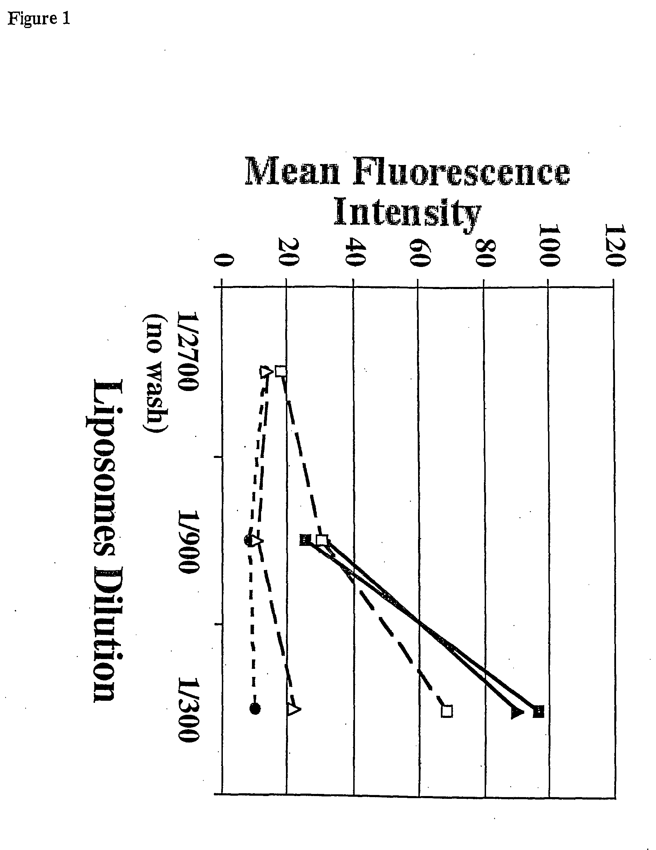

### Figure 1 : Titration of DNP-liposomes binding to human dendritic cells.

The titration of DNP-liposomes binding to dendritic cells was implemented by incubating on ice 3.10⁵ DCs, DNP bearing fluorescent liposomes at different dilutions (1/300 to 1/2.700) and anti-DNP monoclonal antibodies (U7.27.7, and U7.6.3) or irrelevant mouse antibodies in PBS containing 2 mg/ml human albumin. After washing, the mean fluorescence intensity associated to the cells was measured by flow cytometry for the different conditions.

The X-axis of the figure refers to the liposomes dilution, the Y-axis refers to the mean fluorescence intensity measured. The binding of liposomes to DCs in presence of an irrelevant mAb is indicated by dark circles. Triangles correspond to liposomes coated with anti-DNP antibodies of IgG2a isotype (U7.27.7) at 5 µg/ml (dark triangles) or 0,5 µg/ml (clear triangles). Squares correspond to liposomes coated with anti-DNP antibodies of IgG1 isotype (U7.6.3) at 5 µg/ml (dark squares) or 0,5 µg/ml (clear squares).

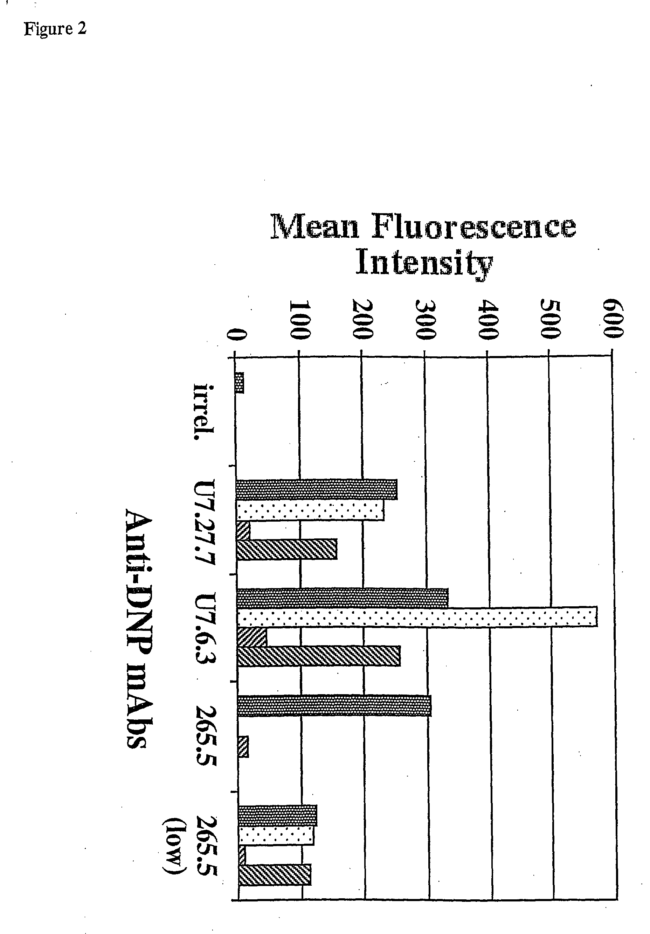

### Figure 2 : Blocking FcγR inhibits binding to human dendritic cells.

DCs, anti-FcR mAbs (at 100 µg/ml), DNP bearing fluorescent liposomes and anti-DNP antibodies (U7.27.7, U7.6.3 and 265.5 at 5 µg/ml, and also at 1 µg/ml for 265.5) were incubated on ice for one hour. After washings, the mean fluorescence intensity associated to the cells was measured by flow cytometry for the different conditions.

The X-axis of the figure refers to the different anti-DNP mAbs, the Y-axis refers to the mean fluorescence intensity measured. On the X-axis, the dark bars correspond to the absence of blocking, the clear bars correspond to the blocking with mouse IgG, the left hatched bars correspond to blocking with anti-FcγR II monoclonal antibody IV.3, the right hatched bars correspond to blocking with anti-FcγR III mAb 3G8.

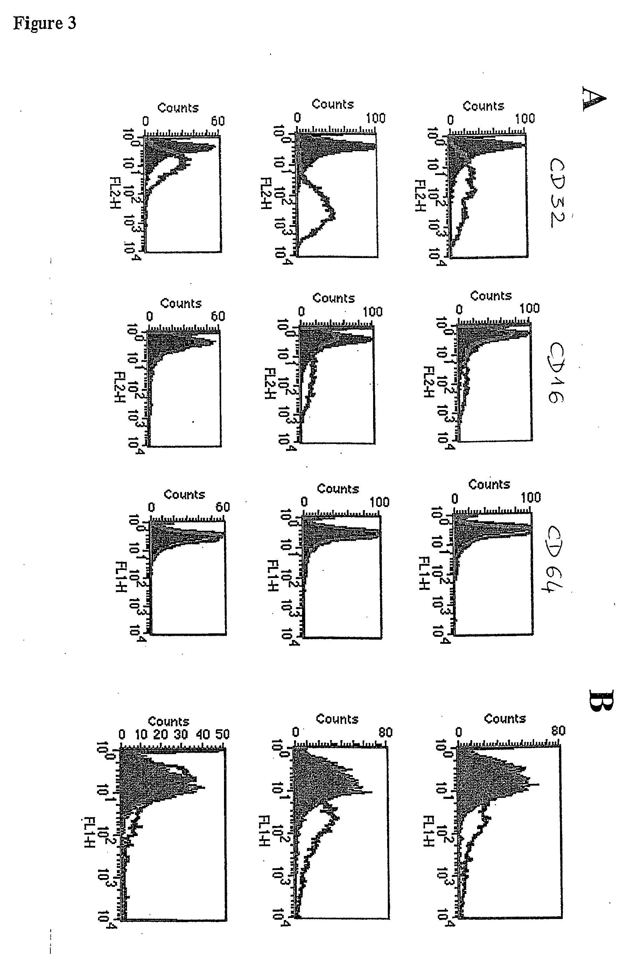

### Figure 3 : Modulation of CD32 affects liposome binding.

Day 7 elutriated DCs were treated for 40 hours with dexamethasone 10⁻⁶ M or with anti-CD40 monoclonal antibodies (3 µg/ml), in presence of GM-CSF 500 U/ml and IL-13 50ng/ml in AIM V medium. The cells were then harvested, then immunophenotyping and binding experiments were performed.

The figure 3A represents the immunophenotyping of DCs treated with dexamethasone, anti CD-40 monoclonal antibodies or without treatment, for their CD32, CD16 and CD64 expression. The upper, intermediary and lower lines of graphs correspond respectively to the DCs without any treatment, DCs treated with dexamethasone and DCs treated with anti CD40 monoclonal antibodies. The left, central and right columns of graphs correspond respectively to the detection of the CD32, CD16, and CD64 expression. In each test, the clear histogram represents the binding of the anti-CD16, anti-CD32 or anti-CD64 antibodies to the DCs. The dark histogram represents the binding of an irrelevant control antibody.

The figure 3B represents the binding of DNP-liposomes to the DCs without treatment (upper graph), DCs treated with dexamethasone (intermediary graph) and DCs treated with anti-CD40 antibodies (lower graph). The dark histogram represents the binding of DNP-bearing fluorescent liposomes to the DCs in the presence of an irrelevant antibody, the clear histogram represents the binding of DNP-bearing fluorescent liposomes to the DCs in the presence of the anti-DNP mouse monoclonal antibody U.7.6.3.

### Example I : Targeting of an exogenous antigen to human antigen presenting cells.

In the example, the abbreviations have the following meaning :

DCs: dendritic cells, CD: cluster of differentiation, IgG: immunoglobulin G, FcγR: receptor for the Fc portion of IgG, DNP: dinitrophenyl, mAb: monoclonal antibody, PBS: phosphate buffered saline, Ag: antigen, GM-CSF: Granulocyte-Macrophage Colony Stimulating Factor, FACS: fluorescence activated cell sorter, IL: interleukin, RPMI: Rosewell Park Memorial Institute, AIM : Adoptive Immunotherapy Media, IFN: interferon, PE: phycoerythrin, FITC: Fluorescein Isothiothiocyanate, PHA : phytohemagglutinin

### Cells, antibodies and liposomes

Antigen presenting cells are elutriated human DCs, prepared according EP 97 924 012.4 and Boyer et al., 1999 ("Generation of phagocytic MAK and MAC-DC for therapeutic use: Characterization and in vitro functional properties" Exp. Hematol. 1999, 27, 751-761). Immunophenotyping of the human immature dendritic cells shows, in average, expression of IgG surface receptor FcγRI (CD64) at a very low level (lower than 10% of positive cells, at most 10 Mean Fluorescence Intensity), FcγR III (CD16) at a low level (lower than 30% of positive cells, at most 50 Mean Fluorescence Intensity) and FcγR II (CD32) at a high level (more than 50% of positive cells, at least 150 Mean Fluorescence Intensity).

U7.27.7 (IgG2a) and U7.6.3 (IgGl) are mouse anti-DNP monoclonal antibodies (mAb) provided by Zelig Eshhar (Weizmann Institute, Rehovot, Israel). Anti-DNP mAb 265.5 (IgG1) is also used (Immunotech).

Liposomes (80 µmoles with respects to lipids) are made from 65% (mol/mol) dimyristoyl phosphatidylcholine, 34,5% cholesterol (Sigma-Aldrich) and 0,5% DNP-caproyl-phosphatidylethanolamine (DNP-cap PE) (Molecular Probes). Liposomes are formed by exposing lipids evaporated from chloroform/methanol (9:1 v/v) to an aqueous solution containing 10 mM carboxyfluorescein (Molecular probes) or 10 mM carboxyfluorescein and influenza proteins containing determinants which may be presented in the context of class II and class I MHC molecules (for instance Mutagrip Vaccine (Institut Pasteur))in PBS. Following repeated cycles of freezing and thawing, liposomes are formed by extrusion (Extruder, Lipex biomembranes, Vancouver, Canada) through polycarbonate filters of 200 nm pore size at 40°C, followed by gel filtration over Sepharose 4B columns to eliminate unencapsulated solute. Laser light scattering determinations indicate that these preparations are homogenous with diameters closely corresponding to the pore size of the polycarbonate filters used (data not shown). Lipid vesicles are sterilized by filtration through 0,45 µm filters. The amount of liposome-associated Ag used for presentation experiments is obtained by serial ten fold dilutions of these liposomes. The final concentration of influenza proteins encapsulated is 1,6 µg/ml. Anti-DNP monoclonal antibodies are used to target DNP-bearing liposomes to the FcR.

### Titration of liposomes binding to DCs

Titration of DNP-liposomes binding to DCs is implemented by incubating on ice for one hour 3.10⁵ DCs, DNP bearing fluorescent liposomes at different dilutions (1/300 to 1/2.700) and anti-DNP monoclonal antibodies (U7.27.7, U7.6.3) or irrelevant monoclonal antibodies, in PBS containing 2 mg/ml human albumin. After washing, the mean fluorescence intensity associated to the cells was measured by flow cytometry for the different conditions.

### Blocking of FcγR binding

Monoclonal antibody IV.3, an anti-FcγR II antibody of IgG2b isotype, and monoclonal antibody 3G8, an anti-FcγR III antibody of IgG1 isotype, were both given by Medarex Company.

DCs, anti-FcR mAbs (at 100 µg/ml), DNP bearing fluorescent liposomes and anti-DNP antibodies (U7.27.7, U7.6.3 and 265.5 at 5 µg/ml, and also at 1 µg/ml for 265.5) were incubated on ice for one hour. After washings, the fluorescence associated with the cells was measured by flow cytometry for the different conditions.

### Modulation of CD32 expression

Day 7 elutriated DCs were treated for 40 hours with Dexamethasone 10⁻⁶ M or with anti-CD40 monoclonal antibodies (3 µg/ml), in presence of GM-CSF 500 U/ml and IL-13 50 ng/ml in AIM V medium. The cells were harvested, and immunophenotyping and binding experiments were performed.

### Flow cytometry

3.10⁵ DCs incubated with DNP fluorescent liposomes and anti-DNP monoclonal antibodies were washed and resuspended in PBS containing 3 nM of the nucleic acid stain TO-PRO-3 (Molecular Probes, Eugene, OR) to exclude dead cells from analysis.

For FcR staining, 3.10⁵ DCs in PBS containing 2 mg/ml human albumin were incubated on ice for 30 min with PE-conjugated monoclonal antibody anti-CD32, PE-conjugated monoclonal antibody anti-CD16, or FITC-conjugated monoclonal antibody anti-CD64 (Immunotech, Marseille, France), then washed and resuspended in PBS containing 3 mM TO-PRO-3.

Flow cytometry analysis was performed in a FACSCalibur with a CellQuest software (BDIS, San Jose, CA).

### Ag presentation assay:

CD4+ T cells are obtained from monocyte-depleted total lymphocytes by negative selection with CD8 and CD19 Midi MACS magnetic beads (Miltenyi biotec). CD8+ T cells are obtained by negative selection with CD4 and CD19 magnetic beads. The cells are then stimulated one or two times with autologous elutriated DCs pulsed with influenza proteins containing determinants which pay be presented in the context of class II and class I MHC molecules. For the first stimulation, 2,5.10⁵ influenza proteins pulsed DCs/ml were incubated with 2,5.10⁶ T cells/ml in complete RPMI additioned with 10% AB+ serum, IL-6 1000 U/ml and IL-12 5 ng/ml. After 7 days, T cells were restimulated with the same number of thawed autologous influenza proteins pulsed DCs in complete RPMI additioned with 10% AB+ serum, IL-2 20 U/ml and IL-7 10 ng/ml.

The TcR-dependent immunostimulation of T lymphocytes reacting to the antigen presented by the APCs is performed as follows. Elutriated DCs are incubated overnight in AIM V additioned with GM-CSF 500 U/ml and IL-13 50 ng/ml with different concentrations of influenza proteins, free or encapsulated in DNP liposomes, in presence of 5 µg/ml of anti-DNP monoclonal antibody or an irrelevant monoclonal antibody. After washing, DCs are incubated with primed autologous CD4+ or CD8+ T cells (see above) and IFNγ producing cells are detected by ELISPOT. Briefly, 5.10⁴ DCs were incubated for 40 hours at 37°C 5% CO₂ with T cells (10³ to 10⁵, in a total volume of 200 µl of RPMI additioned with 10% AB+ serum) in a 96 well nitrocellulose plate precoated with anti-IFNγ monoclonal antibody (Mabtech), then the plate is washed and a second biotynilated anti-IFNγ monoclonal antibody (Mabtech) is added. Vectastain ABC kit (Vector, Burlingame, CA) is used for detection of the spots. All the assays are done in duplicate. Positive controls include T cells stimulated with PHA (Sigma) and ionomycin (Sigma), negative controls include unstimulated T cells or T cells incubated with autologous unpulsed DCs.

### Results

DNP-liposomes binding to DCs is detectable by flowcytometry 1/300 dilution of liposomes after incubation at 4°C (Figure 1) or at 37°C of DCs, DNP-bearing liposomes and anti-DNP antibodies. At the microscope, DC incubated at 37°C with green fluorescent liposomes appear diffusely green, although after incubation at 4°C with the same fluorescent liposomes they show a phenomenon of "capping", with only small peripherical areas of the cells coloured in green. These results indicate efficient internalisation of liposomes by the DCs at 37°C.

The specificity of the binding of DNP-liposomes to the antigen presenting cells was assessed by incubating the immunocomplexes constituted by fluorescent liposomes and anti-DNP antibodies, with the DCs in presence of different anti- FcγR blocking antibodies. Results shown in Figure 2 show that anti-FcγR II antibody mAb IV.3 is the most efficient blocker of the binding of the opsonized micro particle complexes to the cells. Therefore, the binding of liposomes to the DCs via anti-DNP antibodies from different isotypes, is mainly mediated by the CD32 Fcγ receptor expressed on these antigen presenting cells.

The incubation of these human DCs, having internalized and processed the opsonized micro particle complexes, with autologous peripheral blood lymphocytes induces the proliferation of specific CD4 and CD8 T lymphocytes.

The modulation of the expression of CD32 exerts an effect on the binding of DNP-liposomes to the cells, as shown in Figure 3. In fact, treatment of DCs with dexamethasone 10⁻⁶ M upregulates the expression of the CD32 on the surface of the cells, which is correlated with an increased binding of DNP-liposomes to the cells. On the contrary, treatment of DCs with anti-CD40 mAb down regulates the expression of CD32, and results in a lower binding of DNP-liposomes to the cells. This experiment further confirms the specificity of the opsonized micro particle complexes targeting FcγR II of the antigen presenting cells.

The antigen presentation assays show that influenza proteins derived peptides are presented by DCs to CD4+ T cells and to CD8+ T cells.

In these experiments, the peptides are more efficiently presented to CD4+ and CD8+T cells when the influenza proteins derived peptides are encapsulated in targeted liposomes than when they are encapsulated in non targeted liposomes or when free in solution.

### Example II : Preparation of destabilised endocytic vesicles of immature dentritic cells to make them capable to present class-I associated antigens.

Virosomes, which may be considered as liposomes containing viral components, contain influenza hemagglutinin and DNP-caproyl-phosphatidylethanolamine in the membrane ; they also encapsulate the antigen of interest.

Virosomes are prepared and purified by sucrose density gradient according to Schoen et al. (Schoen P., Leserman L. & Wilshut J. "Fusion of reconstituted influenza virus envelopes with liposomes mediated by streptavidin/biotin interactions" FEBS Letters, 1996, 390, 315-318) and Stegmann et al. (Stegmann T., Morselt H.W.M. , Booy F.P., Van Breemen J.F.L., Scherphof G. & Wishut J., 1987, EMBO J., 6, 2651-2659). The protocol of the antigen encapsulation in virosomes is similar to example 1.

Immature dendritic cells are treated with neuraminidase to remove sialic acid residues. Then, the dendritic cells are incubated in the cold with virosomes, in the presence of anti-DNP antibodies. Since the flu hemagglutinin uses sialic acid residues to bind to the cell, in the absence of the sialic acid, the binding in this instance is solely mediated by the anti-DNP antibodies binding to the DNP on the virosomes and the Fc receptor on the dendritic cells. When the cells are brought to 37°C, the virosomes are internalised and the hemagglutinin fusion activity, which has been shown as being independent of its binding activity, permits entry of the virosomes contents into the cytosol as a consequence of the acid pH of the endocytic vesicles.

Thus, the antigen previously encapsulated on the virosome is released into the cytosol.

### Antigen presentation assay

CD4+ and CD8+ T cells are obtained from monocyted-depleted total lymphocytes as described in example I, then stimulated one or two times with autologous elutriated DCs pulsed with the antigen. The immunostimulation of T lymphocytes reacting to the antigen presented by the APCs is performed as described in example 1.

DNP-virosomes binding to DCs occurs after incubation of DCs, DNP-bearing virosomes and anti-DNP antibodies. The incubation of the human DCs, having internalized and processed the opsonized micro-particle complexes, and the corresponding peripheral blood lymphocytes induces the proliferation of specific CD4+ and CD8+ T lymphocytes.

The antigen presentation assays show that the antigen derived peptides are presented by DCs to CD4+ T cells and to CD8+ T cells. In these experiments, the peptides are more efficiently presented to T cells by at least 2 log 10 when the antigen derived peptides are encapsulated in targeted virosomes than when they are free in solution.

## Claims

1. Opsonized micro-particle complex comprising :
- a micro-particular vector encapsulating at least one antigen
- and at least one antibody or fragment thereof,
with said antibody being a human or humanized antibody or an antibody binding to human **FcR** with substantially the same affinity and avidity as the ones of a human antibody and with said antibody or fragment thereof having the carboxy terminal end of its Fc portion external with respect to the opsonized micro particle complex.

2. Opsonized micro particle complex according to claim 1, wherein the antibody is bound to the micro-particular vector through its variable portion.

3. Opsonized micro particle complex according to claim 1 or 2, wherein the micro-particular vector encapsulating the antigen bears determinants and the antibody is bound to the micro-particular vector through its variable portion specific of said determinants.

4. Opsonized micro particle complex according to claim 1, wherein the antibody is directly linked to the micro-particular vector by its Fc portion, with the carboxy terminal end of the Fc portion being external with respect to the opsonized micro particle complex.

5. Opsonized micro particle complex according to any one of claims 1 to 4, wherein the micro-particular vector is a liposome or a micro particle, advantageously having a size of about 20 to about 1000 nm.

6. Opsonized micro particle complex according to anyone of claims 1 to 5, wherein the micro-particular vector contains at least one destabilizing agent for the membrane of the endocytic vesicle, for the improvement of the delivery of the antigen contained in said micro-particular vector, with said destabilizing agent being, for instance, proteins, peptides or lipids of viral or synthetic origin.

7. Opsonized micro particle complex according to any one of the claims 1 to 6, wherein the determinant is a peptide, a polypeptide, a sugar molecule, DNP or another determinant.

8. Opsonized micro particle complex according to any one of claims 1 to 7, wherein the antibody is a human or humanized antibody.

9. Opsonized micro particle complex according to any one of the claims 1 to 8, wherein the antigen is a tumor antigen or an antigen relevant in auto immune or infectious diseases or an allogenic antigen and preferably a combination of tumor antigens.

10. Opsonized micro particle complex according to any one of the claims 1 to 9, wherein the antigen-presenting cell is a dendritic cell, and particularly monocyte-derived immature dendritic cells.

11. Combined preparation containing as active substance the following individual components, in the form of a kit of parts :
- a micro-particular vector encapsulating at least one antigen
- at least one antibody or fragment thereof, with said antibody being a human or humanized antibody or an antibody binding to human FcR with substantially the same affinity and avidity as the ones of a human antibody, and being liable to bind to said micro-particular vector, in such a way that the antibody or fragment thereof has the carboxy terminal end of its Fc portion which remains free,
- possibly human antigen presenting cells bearing Fc receptors, liable to bind to the above-mentioned free Fc portion of the antibody or fragment thereof, for the simultaneous, separate or sequential use, in the vaccination against cancer, infectious or autoimmune diseases.

12. Ternary complex between the opsonized micro particle complex according to any one of claims 1 to 10, and a human antigen presenting cell bearing Fc receptors, wherein the opsonized micro particle complex is bound to the antigen presenting cell Fc receptor through the carboxy terminal end of Fc portion of the antibody.

13. Use of an opsonized micro particle complex, according to any one of claims 1 to 10, or of a ternary complex according to claim 12 as a drug, particularly as a vaccine.

14. Vaccine comprising as active substance an opsonized micro particle complex according to any one of claims 1 to 10, or a ternary complex according to claim 12, possibly in association with a pharmaceutically acceptable vehicle.

15. Use of an opsonized micro particle complex according to any one of claims 1 to 10, or of a ternary complex according to claim 12, for the preparation of a vaccine against cancer, infectious or auto-immune disease.

16. Method for ***in vitro***, or ***ex vivo*** targeting antigens to human antigen presenting cells allowing antigen presentation via MHC class I pathway, comprising the step of contacting an opsonized micro particle complex according to any one of claims 1 to 10, with human antigen presenting cells, to form a ternary complex between the opsonized micro particle complex and said human antigen presenting cells.

17. Method for ***in vitro***, **or *ex vivo*** targeting antigens to human antigen presenting cells allowing antigen presentation via MHC class I pathway, comprising the step of contacting a micro-particular vector encapsulating at least one antigen, at least one antibody or fragment thereof, with said antibody being a human or humanized antibody or an antibody binding to human FcR with substantially the same affinity and avidity as the ones of a human antibody and liable to bind to the micro-particular vector in such a way that the carboxy terminal end of its Fc portion is external with respect to the opsonized micro-particle complex, and human antigen presenting cells, to form a ternary complex between the micro-particular vector, the antibody and the human antigen presenting cells.

18. Method according to any one of the claims 16 or 17, wherein the antigen presentation via MHC class II is also involved.

19. Method according to any one of the claims 16 to 18, wherein the stimulation of human CD8 + T cells specific for exogenous antigen is involved.
